# EUROPEAN PATENT APPLICATION

(11) **EP 1 923 055 A1**
(43) Date of publication of application: **21.05.2008**
(21) Application number: 06121302.1
(22) Date of filing: 26.09.2006
(51) Int. Cl.: A61K 9/20, A61K 31/135, A61P 37/06

(54) **Freeze-dried pharmaceutical composition comprising an S1P agonist or modulator**

(71) Applicant: Novartis AG, 4056 Basel (CH)
(72) Inventor: The designation of the inventor has not yet been filed
(74) Representative: Vögeli-Lange, Regina

(57) **Abstract**

The present invention provides a rapid disintegrating pharmaceutical composition comprising a freeze dried dosage form of an S1P agonist or modulator.

## Description

The present invention relates to pharmaceutical compositions comprising a sphingosine-1 phosphate receptor agonist and/or modulator.

Sphingosine-1 phosphate (hereinafter "S1P") is a natural serum lipid. Presently there are 8 known S1P receptors, namely S1P1 to S1P8. S1P receptor agonists have accelerating lymphocyte homing properties.

S1P receptor agonists are immunomodulating compounds which elicit a lymphopenia resulting from a re-distribution, preferably reversible, of lymphocytes from circulation to secondary lymphatic tissue, evoking a generalized immunosuppression. Naive cells are sequestered, CD4 and CD8 T-cells and B-cells from the blood are stimulated to migrate into lymph nodes (LN) and Peyer's patches (PP), and thus infiltration of cells into transplanted organs is inhibited.

The various known S1P receptor agonists show structural similarities, which result in related problems in providing a suitable formulation. In particular, there is a need for an S1P receptor agonist containing formulation which is well-adapted for oral administration in a solid form, e.g. as a tablet or capsule.

The oral route is often the most convenient route for drug administration, but unfortunately many patients have difficulties in swallowing or no water is available at the time of ingestion.

This can be overcome by using rapid disintegrating tablets hence improving compliance by reducing or masking an unpleasant taste.

Accordingly, the present invention provides a rapid disintegrating solid pharmaceutical composition suitable for oral administration, comprising a freeze dried dosage form of an S1P receptor agonist or modulator.

The present invention therefore provides rapid dispersing dosage forms which disintegrate rapidly in the mouth and which do not depend on the use of sweetening or flavoring agents to mask the taste nor do they depend on the presence of a liquid for washing down the dosage form.

The compositions of the present invention are capable of disintegrating in the mouth, in particular in saliva.

Preferably, the dosage forms have good mouth feel and do not exhibit premature release of the drug in the mouth.

In one embodiment of the present invention, the compositions comprise a freeze-dried dosage form comprising an S1P agonist/modulator particles which may be uncoated or coated with a polymer or lipid material which exhibit minimal release of the drug in the mouth.

This may be achieved, for example, by using coarse coated drug particles and controlling the viscosity of the suspension by reducing the temperature during the holding time in suspension to minimize sedimentation of the particles without altering the physical properties of the dried units.

The resulting dosage form exhibits delayed release of the drug for a time at least sufficient to mask the taste in the mouth before swallowing, and typically for a longer period of time to provide controlled or sustained release of the drug after swallowing.

The carrier material, which forms a network or matrix containing the S1P agonist and/or modulator, after freeze drying, may be any water-soluble or water-dispersible material that is pharmaceutically acceptable, inert to the pharmaceutically active substance and which is capable of forming a rapidly disintegrating network, i.e. disintegrates within, for example 10 seconds or less in the mouth.

The effect of the freeze dried dosage form is that the dosage form is highly dispersed and as a consequence is able to disintegrate rapidly. As a result the compositions may form fine suspensions or solutions on contact with saliva in the mouth.

The preferred carrier material for use in the present invention is gelatin, usually pharmaceutical grade gelatin. Other substances may be used as the carrier material are, for example, hydrolyzed dextrose, dextran, dextrin, maltodextrin, alginates, hydroxyethyl cellulose, sodium carboxymethyl cellulose, microcrystalline cellulose, corn-syrup solids, pectin, carrageenan, agar, chitosan, locust bean gum, xanthan gum, guar gum, acacia gum, tragacanth, conjac flower, rice flower, wheat gluten, sodium starch glycolate, soy fiber protein, potato protein, papain, horseradish peroxidase, glycine and mannitol.

The oral solid rapidly disintegrating composition of the present invention comprising a freeze-dried dosage form of an S1P agonist or modulator may also comprise additional excipients, which may be, for example a cellulose or a sugar alcohol.

Additional excipients, where not in use as a carrier material may nevertheless be used and may be selected from for example, sugar alcohols, for example, mannitol, sorbitol, dextrose, sucrose, lactose, maltose, sorbitol, maltodextrins, corn syrup solids, trehalose, polyvinyl pyrrolidone, polyelectrolyte gel A chondroitin sulfate, cellulose, starch derivatives, Pullulan, glycine, docusate Na, PVC, HPC-SL,mannitol & glycerol, gum xanthan/carragean/acacia/guar/tragacanth, mannitol, polysorbate 60, sodium dodecylsulfate, fatty acids, bile salts, sodium methylhydroxybenzoate, sodium propylhydroxybenzoate, polyols, and starch.

The composition may be held within a hydrophilic matrix which gradually or rapidly dissolves in the presence of body fluid.

The composition may additionally include materials which swell on contact with aqueous liquids, and which may be included in the composition, include polymer materials include from cross-linked sodium carboxymethylcellulose, cross-linked hydroxypropylcellulose, high molecular weighthydroxypropylcellulose, carboxymethylamide, potassium methacrylatedivinylbenzene copolymer, polymethylmethacrylate, cross-linked polyvinylpyrrolidone and high molecular weight polyvinylalcohols.

In one embodiment of the present invention, the composition comprises gelatin and a polysaccharide, e.g. Pullulan or a sugar alcohol and a freeze dried dosage form of an S1P receptor agonist or modulator.

In a particular embodiment, the sugar alcohol acts as a structure forming agent.

In another embodiment, the gelatin and the sugar alcohol are present in a ratio of from 3:1 to 1:3, for example 2:1 to 1:2, typically 1:1.

In one example of an embodiment of the present invention, the gelatin is present in an amount of 2 to 10%, for example 2-4% and the sugar alcohol is present in an amount of 0.1 to 15%, for example 0.5 to 8%.

The composition may also include, or alternatively include, binders such as PVP e.g. cellulose, polyethylene glycols, polyvinylpyrrolidone, starch mucilage, acacia, alginic acid, carboxymethylcellulose, hydroxyethylcellulose, hydroxypropylcellulose, hydroxypropylmethylcellulose, dextrin, ethylcellulose, gelatin, glucose, guar gum, hydroxypropylmethylcellulose, magnesium aluminium silicate, kaltodectrin, methylcellulose, polyethylene oxide, povidone, sodium alginate and hydrogenated vegetable oils.

The composition may also include, or alternatively include, disintegrants (with or without effervescent agents), e.g. cross-linked sodium carboxymethyl cellulose (Crosscarmellose), crosspovidone, sodium starch glycolate.

The compositions may also include, or alternatively include, lubricants e.g. stearic acid, magnesium stearate, calcium stearate, zinc stearate, glyceryl palmitostearate, sodium stearyl fumarate, canola oil, hydrogenated vegetable oil such as hydrogenated castor oil (e.g. Cutina® or Lubriwax® 101), mineral oil, sodium lauryl sulfate, magnesium oxide, colloidal silicon dioxide, polyethylene glycol, polyvinyl alcohol, sodium benzoate, talc, poloxamer, or a mixture of any of the above.

The composition may also include, or alternatively include, surfactants, e.g. sodium lauryl sulphate, docusate sodium.

The core tablet compositions may also include, or alternatively include, glidants, e.g. silica.

The composition may also include, or alternatively include, flavoring agents.

The composition may also include, or alternatively include, gas producers, e.g. sodium bicarbonate, citric acid.

The composition may also include, or alternatively include, sweeteners.

The composition may also include, or alternatively include, pH adjusting agents, e.g. citric acid, fumaric acid.

The composition may also include, or alternatively include, viscosity enhancers.

In one aspect of the present invention, the rapid disintegration of the solid pharmaceutical composition increases the solubility of the active ingredient(s), for example sphingosine 1 phosphate (S1P) receptor agonists or modulators. In particular, this may be the case in saliva, leading to better solubility of the drug than in the small intestine.

Compositions of the present invention may be in the form of, for example, tablets, capsules, caplets, lozenges, pills, mini-tablets, pellets, beads and granules.

Where the solid composition is in the form of pellets or granules, these may, after application of the coating as described hereinafter, be used as such or to fill capsules, e.g. hard gelatine capsules or other storage means, for example sachets prior to administration.

Pellets and granules may be from 2mm to 0.3mm in diameter, for example, a "normal pellet" has a size of 1-0.6mm and a "bead pellet" has a size of 0.4 to 0.8mm
In one embodiment of the present invention, there is provided a capsule containing a plurality of pellets having a rapid disintegration rate according to the present invention.

Rapid disintegration, or more efficient may provide higher solubility of the active substance. Higher solubility of the drug may lead to a higher bioavailability since the risk of precipitation in the body liquid is lower.

In one embodiment of the present invention, the oral composition described herein promotes the absorption and distribution of the S1P agonist and/or modulator through the blood brain barrier and into the brain.

The term "rapid disintegration" as used herein means that the solid dosage form will disintegrate in water at 37.degree. C. in 60 seconds or less. The forms usually disintegrate in about 5-20 seconds, more usually 5 to 10 seconds or less, when tested by the following procedure which is analogous to the Disintegration Test for Tablets, B.P. 1973 which is described in British patent number 1548022.

The bioavailability of these sphingosine 1 phosphate (S1P) receptor modulators may be improved by adding the buccal absorption site to the oral absorption site potentially leading to decrease the first-pass effect. If sphingosine 1 phosphate (S1P) receptor modulators get buccally absorbed through either the sublingual route, the oral mucosa, the esophageal lining and/or the tonsils, bioavailability would be increased as the buccal absorption route circumvents the Gl tract (p-gp in the gut) and the first pass liver effect. An increased bioavailability may allow to lower the dose leading to an improved safety profile.

Pharmaceutical dosage forms adapted to supply the medicine to the oral cavity for buccal, sublingual or gingival absorption will be used with and without the presence of enhancer agents such as, but not limited to, those described in the examples.

Examples of these dosage forms are the following but not limited to: buccal spray, effervescent tablets, granules, orally disintegrating tablets, thin films or wafers and mucoadhesive discs or patches.

S1P receptor agonists are typically sphingosine analogues, such as 2-substituted 2-aminopropane-1,3-diol or 2-amino-propanol derivatives. Examples of appropriate S1P receptor agonists are, for example:
Compounds as disclosed in EP627406A1, e.g. a compound of formula I 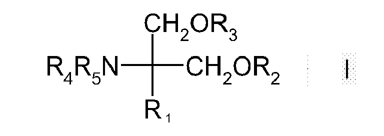
wherein
R₁ is a straight- or branched (C₁₂₋₂₂) carbon chain, which may have in the chain a bond or a hetero atom selected from a double bond, a triple bond, O, S, NR₆, wherein R₆ is H, alkyl, aralkyl, acyl or alkoxycarbonyl, and carbonyl; and/or which may have as a substituent alkoxy, alkenyloxy, alkynyloxy, aralkyloxy, acyl, alkylamino, alkylthio, acylamino, alkoxycarbonyl, alkoxycarbonylamino, acyloxy, alkylcarbamoyl, nitro, halogen, amino, hydroxyimino, hydroxy or carboxy; or
R₁ is a phenylalkyl wherein alkyl is a straight- or branched (C₆₋₂₀)carbon chain; or a phenylalkyl wherein alkyl is a straight- or branched (C₁₋₃₀)carbon chain wherein said phenylalkyl is substituted by
   a straight- or branched (C₆₋₂₀)carbon chain optionally substituted by halogen,
   a straight- or branched (C₆₋₂₀)alkoxy chain optionally substitued by halogen,
   a straight- or branched (C₆₋₂₀)alkenyloxy;
   phenylalkoxy, halophenylalkoxy, phenylalkoxyalkyl, phenoxyalkoxy or phenoxyalkyl;
   cycloalkylalkyl substituted by a straight- or branched (C₆₋₂₀)alkyl chain;
   heteroarylalkyl substituted by a straight- or branched (C₆₋₂₀)alkyl chain;
   heterocyclic alkyl wherein said alkyl is a straight- or branched (C₆₋₂₀)carbon chain;
   or
   heterocyclic alkyl substituted by a straight- or branched (C₂₋₂₀)alkyl chain, and wherein the alkyl moiety may have in the carbon chain, a bond or a heteroatom selected from a double bond, a triple bond, O, S, sulfinyl, sulfonyl, or NR₆, wherein R₆ is as defined above;
and as a substituent alkoxy, alkenyloxy, alkynyloxy, aralkyloxy, acyl, alkylamino, alkylthio, acylamino, alkoxycarbonyl, alkoxycarbonylamino, acyloxy, alkylcarbamoyl, nitro, halogen, amino, hydroxy or carboxy, and each of R₂, R₃, R₄ and R₅, independently, is H, C₁₋₄ alkyl or acyl
or a pharmaceutically acceptable salt thereof;

Compounds as disclosed in EP 1002792A, e.g. a compound of formula II 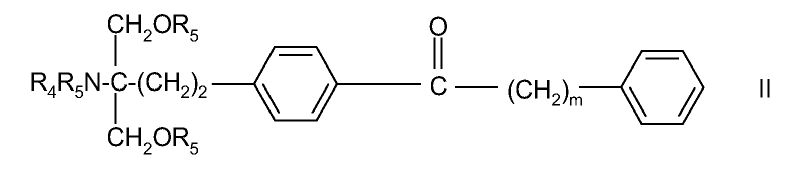 wherein
m is 1 to 9; and
each of R₂, R₃, R₄ and R₅, independently, is H, alkyl or acyl; or a pharmaceutically acceptable salt thereof;

Compounds as disclosed in EP0778263 A1, e.g. a compound of formula III 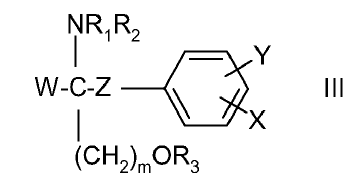 wherein
W is H; straight chain or branched (C₁₋₆)alkyl, (C₂₋₆)alkenyl or (C₂₋₆)alkynyl; unsubstituted or by OH substituted phenyl; R₄O(CH₂)ₙ; or straight chain or branched (C₁₋₆)alkyl substituted by 1 to 3 substituents selected from the group consisting of halogen, cycloalkyl, phenyl or phenyl substituted by OH;
X is H or unsubstituted or substituted straight chain alkyl having a number p of carbon atoms or unsubstituted or substituted straight chain alkoxy having a number (p-1) of carbon atoms, e.g. substituted by 1 to 3 substitutents selected from the group consisting of alkyl, OH, alkoxy, acyloxy, amino, alkylamino, acylamino, oxo, haloalkyl, halogen, unsubstituted phenyl or phenyl substituted by 1 to 3 substituents selected from the group consisting of alkyl, OH, alkoxy, acyl, acyloxy, amino, alkylamino, acylamino, haloalkyl and halogen;
Y is H, alkyl, OH, alkoxy, acyl, acyloxy, amino, alkylamino, acylamino, haloalkyl or halogen, Z is a single bond or a straight chain alkylene having a number or carbon atoms of q,
each of p and q, independently, is an integer of 1 to 20, with the proviso of 6≤p+q≤23, m is 1, 2 or 3,
n is 2 or 3,
each of R₁, R₂, R₃ and R₄, independently, is H, alkyl or acyl,
or a pharmaceutically acceptable salt thereof.
Compounds as disclosed in WO02/18395, e.g. a compound of formula IVa or IVb 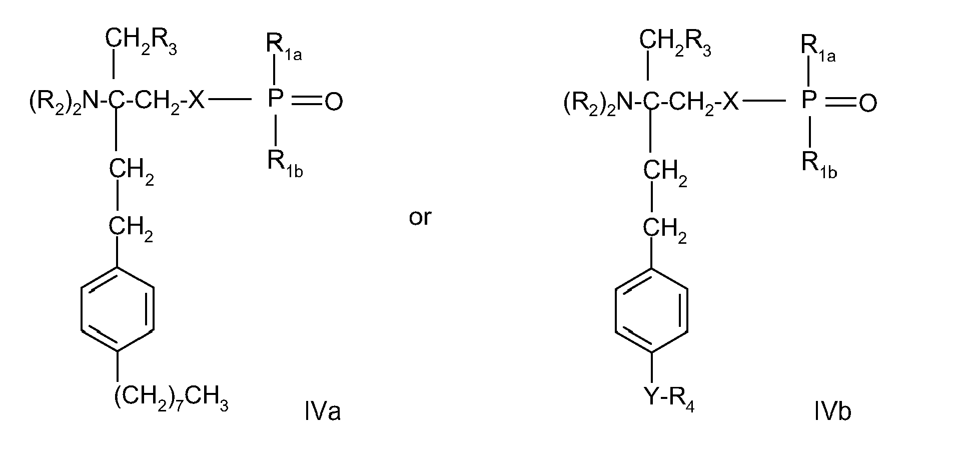 wherein
X is O, S, NR₁ or a group -(CH₂)ₙ-, which group is unsubstituted or substituted by 1 to 4 halogen;
n is 1 or 2,
R₁ is H or (C₁₋₄)alkyl, which alkyl is unsubstituted or substituted by halogen;
R₁ₐ is H, OH, (C₁₋₄)alkyl or O(C₁₋₄)alkyl wherein alkyl is unsubstituted or substituted by 1 to 3 halogen;
R_{1b} is H, OH or (C₁₋₄)alkyl, wherein alkyl is unsubstituted or substituted by halogen;
each R₂ is independently selected from H or (C₁₋₄)alkyl, which alkyl is unsubstituted or substitued by halogen;
R₃ in case of a compound of formula IVa is H, OH, halogen or O(C₁₋₄)alkyl wherein alkyl is unsubstituted or substituted by halogen;
R₃ in case of a compound of formula lVb is H, OH, halogen, (C₁₋₄)alkyl wherein alkyl is unsubstituted or substituted by hydroxy, or O(C₁₋₄)alkyl wherein alkyl is unsubstituted or substituted by halogen,
Y is -CH₂-, -C(O)-, -CH(OH)-, -C(=NOH)-, O or S,
R₄ is (C₄₋₁₄)alkyl or (C₄₋₁₄)alkenyl;
or a pharmaceutically acceptable salt or hydrate thereof;
Compounds as disclosed in WO 02/06268 or JP-14316985, e.g. a compound of formula VII 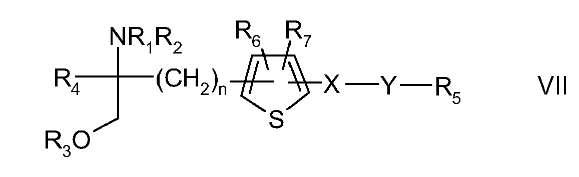 wherein
each of R₁ and R₂, independently, is H or an amino-protecting group;
R₃ is hydrogen or a hydroxy-protecting group;
R₄ is (C₁₋₆)alkyl;
n is an integer of 1-6;
X is ethylene, vinylene, ethynylene, a group having a formula - D-CH₂- (wherein, D is carbonyl, a group having a formula -CH(OH)-, O, S or N; aryl or aryl substituted by three members selected from group a as defined hereinafter;
Y is single bond, C₁₋₁₀alkylene, C₁₋₁₀alkylene which is substituted by one to three substituents selected from groups a and b, C₁₋₁₀alkylene having O or S in the middle or end of the carbon chain, or C₁₋₁₀alkylene having O or S in the middle or end of the carbon chain which is substituted by one to three substituents selected from groups a and b;
R₅ is hydrogen, cycloalkyl, aryl, heterocycle, cycloalkyl substituted by one to three members selected from groups a and b, aryl substituted by one to three members selected from groups a and b, or heterocycle substituted by one to three members selected from groups a and b; and
each of R₆ and R₇, independently, is H or a substituent selected from group a;
<group a> is halogen, lower alkyl, halogeno lower alkyl, lower alkoxy, lower alkylthio, carboxyl, lower alkoxycarbonyl, hydroxy, lower aliphatic acyl, amino, mono-lower alkylamino, di-lower alkylamino, lower aliphatic acylamino, cyano and nitro;
<group b> is cycloalkyl, aryl, heterocycle, each being optionally substituted by up to three substituents selected from group a;
with the proviso that when R₅ is hydrogen, Y is either a single bond or linear C₁₋₁₀ alkylene,
e.g. (2R)-2-amino-4-[3-(4-cyclohexyloxybutyl)benzo[b]thien-6-yl]-2-methylbutan-1-ol,
or a pharmacologically acceptable salt or ester thereof.

When in the compounds of formula I the carbon chain as R₁ is substituted, it is preferably substituted by halogen, nitro, amino, hydroxy or carboxy. When the carbon chain is interrupted by an optionally substituted phenylene, the carbon chain is preferably unsubstituted. When the phenylene moiety is substituted, it is preferably substituted by halogen, nitro, amino, methoxy, hydroxy or carboxy. Acyl may be a residue R-CO-, wherein R is C₁₋₆alkyl, C₃₋₆cycloalkyl, phenyl or phenyl-C₁₋₄alkyl.

Preferred compounds of formula I are those wherein R₁ is a straight or branched, preferably straight, chain alkyl having 13-20 carbon atoms, optionally substituted by nitro, halogen, amino, hydroxy or carboxy, and, more preferably those wherein R₁ is phenylalkyl substituted by a straight or branched C₆₋₁₄alkyl chain optionally substituted by halogen and the alkyl moiety is a C₁₋₆alkyl optionally substituted by hydroxy. More preferably, R₁ is phenyl-C₁₋₆alkyl substituted on the phenyl by a straight or branched, preferably straight, C₆₋₁₄alkyl chain. The C₆₋₁₄alkyl chain may be in ortho, meta or para, preferably in para.

Preferably each of R₂ to R₅ is H.

A preferred compound of formula I is 2-amino-2-tetradecyl-1,3-propanediol. A particularly preferred S1P receptor agonist of formula I is 2-amino-2-[2-(4-octylphenylethyl)]propane-1,3-diol in free form or in a pharmaceutically acceptable salt form (referred to hereinafter as Compound A), e.g. the hydrochloride, i.e. FTY720, as shown:

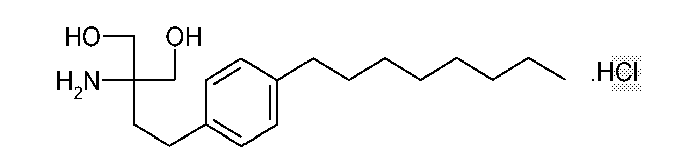

A preferred compound of formula II is one wherein each of R₂ to R₅ is H and m is 4, i.e. 2-amino-2-{2-[4-(1-oxo-5-phenylpentyl)phenyl]ethyl}propane-1,3-diol (referred to hereinafter as Compound B), in free form or in a pharmaceutically acceptable salt form, e.g. the hydrochloride.

A preferred compound of formula IVa is the Compound A-phosphate (R₂ is H, R₃ is OH, X is O, R₁ₐ and R_{1b} are OH). A preferred compound of formula V is Compound B-phosphate (R₁ is CH₂OH, R₃ is H, X is O, m is 1, R₂ is phosphate and R is 2-[4-(1-oxo-5-phenylpentyl)phenyl]ethyl).

When the compounds of formulae I to VII have one or more asymmetric centers in the molecule, the various optical isomers, as well as racemates, diastereoisomers and mixtures thereof are embraced.

Examples of pharmaceutically acceptable salts of the compounds of formulae I to Vll include salts with inorganic acids, such as hydrochloride, hydrobromide and sulfate, salts with organic acids, such as acetate, fumarate, maleate, benzoate, citrate, maleate, methanesulfonate and benzenesulfonate salts, or, when appropriate, salts with metals, such as sodium, potassium, calcium and aluminium, salts with amines, such as triethylamine and salts with dibasic amino acids, such as lysine. The compounds and salts of the present invention encompass hydrate and solvate forms.

The composition of the present invention may comprise one or more salts and/or free acid of the S1 P agonists and//or modulator.

The composition of the invention preferably contains 0.01 to 20% by weight of S1 P receptor agonists, more preferably 0.1 to 10%, e.g. 0.5 to 5% by weight, based on the total weight of the composition.

Preferably, the composition is stable for at least six months at ambient temperature.

Preferably, the active ingredient dose ranges from 0 to 1000 mg, for example 0 to 500mg.

The compositions of the invention may show good stability characteristics as indicated by standard stability trials, for example having a shelf life stability of up to one, two or three years, and even longer. Stability characteristics may be determined, e.g. by measuring decomposition products by HPLC analysis after storage for particular times, at particular temperatures, e.g. 20°, 40° or 60°C.

The pharmaceutical compositions of the present invention may be produced by standard processes, for instance by conventional mixing, granulating, sugar-coating, dissolving or lyophilizing processes. Procedures which may be used are known in the art, e.g. those described in L. Lachman et al. The Theory and Practice of Industrial Pharmacy, 3rd Ed, 1986, H. Sucker et al, Pharmazeutische Technologie, Thieme, 1991, Hagers Handbuch der pharmazeutischen Praxis, 4th Ed. (Springer Verlag, 1971) and Remington's Pharmaceutical Sciences, 13th Ed., (Mack Publ., Co., 1970) or later editions.

The dosage forms of the present invention are manufactured by known means, resulting in suspensions and the like. Liquid suspensions are then poured into discrete units, for example contained within the pockets of a suitable mold. Alternatively, the suspension may be in the form of solid units, for example frozen units or gelled units where the carrier material readily forms a gel. Typically each unit will contain up to 250 mg of the drug, for example 10-100 mg. Unit dosage forms of the drug in rapidly disintegrating form are encompassed by the present invention.

The suspension of the particles in the carrier material is preferably formed into discrete units by introduction into a mold which preferably comprises a plurality of depressions, each of the depressions being of the desired shape and size for the oral dosage form product. The mold preferably comprises a plurality of depressions formed in sheet of a filmic material which may be similar to the material employed conventionally in the blister packaging of pharmaceuticals.

Alternative methods of forming discrete frozen or gelled units of the suspension include solidifying the mixtures in dropwise fashion. For example, the suspension may be passed through one or more holes to form drops, spheres or a spray of small particles which can be solidified by passage through a cold gas or liquid, for example liquid nitrogen. Alternatively, the drops, spheres or spray may be solidified by contact with a chilled liquid which is immiscible with the solution or suspension and which has a density such that the drops either fall through the immiscible liquid as they solidify or float on the surface of the immiscible liquid.

Removal of the continuous phase from the discrete units of the suspension comprising the pharmaceutically active substance is carried out by techniques well known to those skilled in the art. For example, when the discrete units are in a liquid form, they will generally be frozen or gelled prior to drying. The suspension contained within the pockets of a suitable mold is frozen, for example by passing a gaseous cooling medium such as liquid nitrogen over the mold or by inserting the mold into a nitrogen spray freezing chamber. Alternatively, the mold may be cooled by passing the mold over a cold surface. Once the dosage forms have been frozen, the mold may be stored in a cold store prior to drying.

Frozen discrete units may be dried by freeze drying according to techniques which are well known in the art. The continuous phase, for example water, is sublimed in a freeze drying process under a reduced pressure which transforms the solid phase solvent (ice) directly into a vapor. The freeze drying process will generally be carried out in a freeze drying chamber typically operating under a vacuum of 0.1 to 1.0 mBar for a period of time of from 180 to 500 minutes.

In one aspect, the present invention relates to a process for producing a pharmaceutical composition, comprising:
(a) mixing a freeze dried dosage form of an S1 P receptor agonist with a structure forming agent;
(b) producing an aqueous suspension,
wherein the aqueous suspension contains less than 50% solid; and
optionally further conducting a lyophillisation step.

In one embodiment of the method of the present invention, the suspension is cooled to 10 to 20 degrees Celsius, for example 15°C, prior to the a lyophillisation step.

In particular, the pharmaceutical compositions are useful for:
a) treatment and prevention of organ or tissue transplant rejection, for example for the treatment of the recipients of heart, lung, combined heart-lung, liver, kidney, pancreatic, skin or corneal transplants, and the prevention of graft-versus-host disease, such as sometimes occurs following bone marrow transplantation; particularly in the treatment of acute or chronic allo- and xenograft rejection or in the transplantation of insulin producing cells, e.g. pancreatic islet cells;
b) treatment and prevention of autoimmune disease or of inflammatory conditions, e.g. multiple sclerosis, arthritis (for example rheumatoid arthritis), inflammatory bowel disease, hepatitis, etc.;
c) treatment and prevention of viral myocarditis and viral diseases caused by viral mycocarditis, including hepatitis and AIDS.

The invention is, in one embodiment, related to the treatment of inflammatory conditions. In one example, the invention is related to compositions capable of regulating and/or suppressing mast cell activation and secretion for the relief of inflammatory conditions, e.g., in the brain as in multiple sclerosis.

There is also provided a method of protecting multiple sclerosis subjects against neurodegerative brain inflammation, comprising the administration to said subjects the a composition as described herein, for example a composition comprising an S1P agonist and/or modulator.

The composition of the present invention and any concentrate for dilution and pharmaceutical solution made therefrom, may be administered in an amount which is therapeutically effective against a disease or condition which can be treated by administration of the S1 P receptor agonist.

The exact amount of S1P receptor agonist or pharmaceutically acceptable salt thereof to administer can vary widely. The dose may depend on the particular compound, route of administration, the rate of administration, the strength of the particular concentrate or pharmaceutical solution employed, the nature of the disease or condition being treated, and the sex, age and body weight of the patient. The dose may also depend on the existence, nature and extent of any adverse side-effects that may accompany the administration of the concentrate or pharmaceutical formulation. Typically, a dose of 0.5 to 5 mg of S1 P receptor agonist, e.g. Compound A, are administered to children.

The composition of the present invention and any concentrate for dilution and respective pharmaceutical solution may be used in combination with other immunosuppressant(s), steroid(s) such as prednisolone, methylprednisolone, dexamethasone, hydrocortisone and the like, or nonsteroidal anti-inflammatory agent. The administration of a combination of active agents may be simultaneous or consecutive, with either one of the active agents being administered first. The dosage of the active agents of a combination treatment may depend on effectiveness and site of action of each active agent, as well as synergistic effects between the agents used for combination therapy.

The invention will now be described with reference to the following specific embodiments.

### Examples

### Example 1

A rapid disintegrating formulation is prepared, which comprises gelatin (3%), mannitol as structure forming agent (1-5%), sweeteners, flavoring agents.

Gelatin and mannitol are added to the water and heated to 40°C to dissolve. The gelatin/mannitol solution is cooled to 23°C and mixed with the active ingredient, e.g. an S1 P agonist or modulator. The total solid content is less than 50%. The suspension is first cooled to 15°C to prevent sedimentation of the suspension before the start of the lyophilization (coated or uncoated).

### Example 2:

As Example 1, except where the mannitol is replaced with sorbitol.

## Claims

1. A rapid disintegrating pharmaceutical composition comprising a freeze dried dosage form of an S1 P agonist or modulator.

2. The composition of claim 1, additionally comprising one or more of gelatin, mannitol, sorbitol, dextrose, sucrose, lactose, maltose, maltodextrins, corn syrup solids, trehalose, polyvinyl pyrrolidone, polyelectrolyte gel A chondroitin sulfate, cellulose, starch derivatives, Pullulan, glycine, docusate Na, PVC, HPC-SL,mannitol & glycerol, gum xanthan/carragean/acacia/guar/tragacanth, mannitol, polysorbate 60, sodium dodecylsulfate, fatty acids, bile salts, sodium methylhydroxybenzoate, sodium propylhydroxybenzoate, viscosity enhancers, flavoring agents, sweeteners.

3. A composition according to claim 2, wherein the S1P receptor agonist comprises 2-amino-2-[2-(4-octylphenyl)ethyl]propane-1,3-diol or 2-amino-2-{2-[4-(1-oxo-5-phenylpentyl)-phenyl]ethyl}propane-1,3-diol or a pharmaceutically acceptable salt thereof.

4. A composition according to claim 1, 2 or 3, further comprising a lubricant.

5. A composition according to claim 4, wherein the lubricant comprises magnesium stearate.

6. A composition according to any preceding claim, comprising 0.5 to 5% by weight of the S1P receptor agonist.

7. A composition according to claim 4, comprising 1.5 to 2.5% by weight of the lubricant.

8. A composition according to claim 1 or 7, further comprising hydroxypropyl cellulose, hydroxypropylmethyl cellulose or methyl cellulose.

9. A composition according to any preceding claim, in the form of a granule.

10. A composition according to any of claims 1 to 9, in the form of a tablet.

11. A composition according to any of claims 1 to 9, in the form of a capsule

12. A composition according to any preceding claim, wherein the disintegration time is less than 10 seconds.

13. A method of treating a subject in need of immunosuppression, comprising administering to the subject a composition according to claim 1.

14. Use of a pharmaceutical composition according to claim 1 for the preparation of a medicament for the prevention or treatment of organ or tissue transplant rejection, or for the prevention or treatment of an inflammatory or autoimmune disease.

15. A process for producing a pharmaceutical composition, comprising
(a) mixing a freeze dried dosage form of an S1 P receptor agonist with a structure forming agent;
(b) producing an aqueous suspension
wherein the aqueous suspension contains less than 50% solid; and
optionally further conducting a lyophillisation step.

16. A method of protecting multiple sclerosis subjects against neurodegerative brain inflammation, comprising the administration to said subjects the composition of any of claims 1 to 12.
